# EUROPEAN PATENT APPLICATION

(11) **EP 1 840 110 A1**
(43) Date of publication of application: **03.10.2007**
(21) Application number: 05817114.1
(22) Date of filing: 29.11.2005
(51) Int. Cl.: C05F 11/08, A01N 65/00, C12N 1/14

(54) **LIQUID MYCORRHIZAL INOCULANT**

(30) Priority: 08.12.2004 CU 4027704
(71) Applicant: Instituto nacional de ciencias agricolas (INCA), San José de las Lajas 32700 (CU)
(72) Inventor: PEREZ CABRERA, Yuniesky, Habana del Este Ciudad de la habana 1170 (CU); DELL'AMICO RODRÍGUEZ, José Miguel, Habana del Este Ciuda de la habana 11700 (CU); FERNÁNDEZ MARTÍN, FéliICA, Carretera Central, jas 32700 (CU)
(74) Representative: Gil-Vega, Victor
(86) International application number: PCT/CU2005/000010
(87) International publication number: WO 2006/060968

(57) **Abstract**

The technical solution proposed is related with agriculture, soil biotechnology, and specifically with the production and application of arbuscular mycorrhizal fungi, a microorganism that improves the efficiency of plant nutrient absorption, water level, vegetable production, protection against some root diseases, etc. It consists of a liquid product to apply arbuscular mycorrhizal fungi, starting from using an aqueous medium that allows to achieve a mycorrhizal propagule stabilization, which enables soil aggregate development and a checked viability in liquid medium between 12 and 18 months that can be used in irrigation and fertigation systems; it can be applied directly in the field or protected crop greenhouses, besides allowing the improvement of spore dormancy mechanisms and achieving a high concentration level that enables its handling and transportation. By applying this product, mycorrhizal fungi can be managed in crop under large scale irrigation systems, achieving better productive and environmental conditions.

## Description

### Technical field

The present technical solution is related with agricultural sciences, particularly with organic biofertilizer production, and more specifically, for obtaining a liquid mycorrhizal inoculant that can be applied to soil and plants both directly and by means of irrigation systems.

### Previous art

Multiple functions have been attributed to mycorrhizal associations, among them are the improvement of the absorbing root system surface through a remarkable increase of this system, soil toxins tolerance increment, solubilization of certain nutrient elements (N, P, Ca, Mg, Mn, Cu, Zn, B, etc.), resistance to adverse conditions such as: drought, salinity, absorption selectivity, improvement of soil physical properties derived from the formation of stable aggregates and certain protection against root pathogens. The application of this association to agriculture implies a strong environmental impact, because it favours a better balance among the different microorganism populations present in the mycorrhizosphere and rhizosphere of mycorrhizal fungus-plant association and in general a higher protection against the biotic and abiotic stress effects.

The obtaining and later application of inoculants based on arbuscular mycorrhizal fungi in plants have particular characteristics, because they do not grow in axenic media alone, but complete their cycle of life in a host plant roots, so that once concluded the vegetable life cycle (for the production of these inoculants 3 to 4 month-old short cycle plants are used), the root system colonized by the fungus is extracted, as well as the fungal propagules (extramatrical mycelium and spores) developed during the association, as well as the substratum where they have been carried out, constituting all these components a mycorrhizal inoculant on a solid base (Fernandez, F. Chapter 3. Bases Cientifico Técnicas para el manejo de los sistemas micorrizados eficientemente. In: Rivera, Fernández, eds. INCA, Havana. 2003).

In the current production of this kind of inoculant, the following substrata are used (Feldman, F. and Idozak, E. Inoculum production of Vesicular-arbuscular Mycorrhizal Fungi for use in tropical nurseries. In: Methods in Microbiology. Volume 24 ISBN 0-12-521524-X. Eds. Academic Press Limited. 1992; Fernandez, F. *et al,* Author's Certificate 22641. OCPI. 1999):
- Soil
- Soil + Organic Matter
- Organic matter
- Sand
- Clays
- Clays + Sand
- Clays + Organic Matter
- Sand + Organic Matter + Soil
- Nutrient solution in a liquid flow chamber
- Vermiculite
- Pearlet
- Moss peat
- Expanded montmorillonite clay
- Combinations of all these subtracts.

Mycorrhizal products developed from the previously mentioned substrata are directly applied to dressed seed with 10%, according to the total weight of the seed to be put straight to the field (Fernandez, F. *et al.* Author's Certificate 22641. OCPI. 1999) or the direct application of inoculants at a rate of 1 to 2 T. h⁻¹. In the case of seedbed and nursery crops, the application rate should not exceed 1 kg.m², and 10 g/seed, always putting the product below the seed to be sown respectively (Sieverding, E. Vesicular. Arbuscular Mycorrhiza in Tropical Agrosystem. Deutsche Gesellsschaft fur tecniische Zusammenarbeit (GTZ) GMBH, Federal Republic of Germany. 1991. 371 p).

The application of these inoculants by irrigation systems or fertigation is mainly due to the absence of a liquid inoculant that meets the appropriate characteristics, allowing it to support the abrupt changes of the hydrostatic pressure generated in the irrigation tubes, what makes impossible the application by these means.

The production of arbuscular mycorrhizal inoculants has been developed on the basis of applying solid, semisolid to flocculant substrata and on a smaller scale the use of these microorganisms in liquid media.

Regarding solid media, there are some examples of patents that have used several multiplication substrata, such as: US4945059, JP7123979, JP4187081, DE10161443, and DE10221762. These inoculants are unsuitable, since they generally manage big volumes of substrata, spore germination is affected by long dormancy periods, propagule concentration in this product is natural, that is, it cannot be concentrated with at the desired size.

In the case of semisolid inoculants, the following patents have been published: EP0726305, EP0596217, EP0072213, US5120344, EP0495108, EP0475433, US5436218, US3437625, US3557562, EP0023347, US2856380, US3857991, and GB2381264. These products are based on the substrata used like gels, copolymers, clays, in flocculant media. As in the previous case, important volumes are managed in the agricultural application which are not able to overcome the dormancy periods in the spores from mycorrhizal fungi.

The liquid inoculants reported along with patents, are basically related with ectomycorrhiza (CN1420167, US2 004208852) and we have only found a liquid reported by Japanese people (JP4141023), based on the use of a stabilizer (citric acid or its salt) to protect spores.

In general, well-known mycorrhizal products do not have a uniform spore germination and dormancy mechanisms of these fungal species cannot be yet improved, because the substrata on which these inoculants are presented do not confer stressing situations to the propagules that accelerate the germination processes of the spores once inoculated under field conditions.

On the other hand, mycorrhizal inoculants manufactured so far, which have a natural limit of mycorrhizal propagules, have not been reported, because the obtaining procedure does not bear the concentration of a higher quantity of specific propagules. The well-known mycorrhizal product with a higher concentration at present has a spore:mycelium relation of 50 spores.g⁻¹, substratum:65 mg. mycelium⁻¹ substratum (Fernández, F. Chapter 3. Bases Cientifico Técnicas para el manejo de los sistemas micorrizados eficientemente. In: Rivera, Fernández, eds. INCA, Havana. 2003).

The specific glycoprotein of these fungi, called Glomaline, accelerates the formation processes of stable micro-aggregates in the soil, improving their physical structure, increasing the oxygen levels, and enabling a better colonization of the organism. As Glomaline is only excreted in a natural way during the symbiosis, or under the action of a certain medium of excretion, it does not appear excreted to the solid support in the well-known mycorrhizal inoculants, and the levels where it is found in the soil depend on the symbiotic status with the organism, and they cannot rise beyond what this symbiosis allows to.

### Description of the Invention

The main objective of this invention consists of obtaining of a liquid product that permits a massive handling of mycorrhizal fungi, with higher quality and efficiency, so that it can be straight applied in appropriate and profitable doses, especially by located irrigation systems or another's ones.

For obtaining this inoculant, the following steps were developed:
1. Seeding host plants of *Braquiaria decumbens, Sorghum vulgare* or another micotrophic obligatory species of host plants which grow on a reproductive substratum: soil, kaolinitic or montmorillonic clays, pearlet, zepeolite, vermiculite and organic matter (applied according to symbiotic needs).
2. These plants are directly inoculated by placing 10 grams of pure strain of mycorrhizal fungi below the seeds, for their production the following species have been individually used: *Glomus fasciculatum, Glomus clarum, Glomus spurcum, Glomus mosseae*, *Glomus intrarradices, Gigaspora margarita,* and other species of Glomales and Diversisporales, but other species of mycorrhizal fungi can also be used.
3. At the end of the plant life cycle, its top part is removed, and a consistent solid substratum is obtained in a mixture of mycorrhizal propagules (colonized roots, fungal mycelium and resistance spores) and the initial support.
4. Later on, mycorrhizal propagules are extracted by wet decantation methods and sifted through several sieves from a 400 to 40 *u*m, according to the mycorrhizal fungi species in question; the solid fraction gathered in the finest sieve is centrifuged, and then the liquid fraction is decanted with fungal propagules.
5. When all the mycorrhizal components have been separated, these are superficially disinfected with a solution of Chloramine T (2%) during 5 minutes; they are washed three times with sterile distilled water and placed in sulfate of streptomycin during 24 hours. Once ended they are packed in a aqueous mixture containing Sorbitol (between 5 and 10%), Mannitol (2%), Tween 40 (5%), Tween 80 (2%), polyethylen glycol 4000, to apply at a range between 100 and 500 mg.l⁻¹ and agar from 0.8 to 0.1%. In this case, the product can be manufactured up to permissible physical limits of propagule unit.ml⁻¹ of culture medium.
6. The aqueous solution with propagules would be composed by the following elements:
   a) Active components:
      Mycorrhizal propagules: resistance spores, arbuscular extramatrical mycelium, Glomaline and smaller than 40 um roots, colonized with the fungus in question.
   b) Other components:
      * Sorbitol, at a range between 5 and 10%,
      * Mannitol, at a range between 2 and 5%,
      * Tween 40, at a range between 5 and 7%,
      * Tween 80, at a range between 2 and 7%,
      * Polyethylene glycol, applied at a range between 100 and 500 mg.l⁻¹
      * Agar, at a range between 0.8 and 0.1 %.

Once these procedures are concluded, a product is ready to be applied in the field or by means of located irrigation systems, guaranteeing that mycorrhizal propagules, especially spores, are protected from the effects of the superficial osmotic pressure and that once they are applied in water, they are able to break spore dormancy mechanisms and reach a strong colonizing capacity, developing a positive crop answer.

The proposed invention consists of an inoculant that, according its form of production, can be highly infective, because very high concentrations can be reached, with no more limitations than the physical ones that allow this aqueous solution, or the conveniences for its application, achieving concentrations in the order of 1 or 2 million spores. l⁻¹ and 1 and 2 grams of mycelium.l⁻¹.

Moreover, because of the fact that mycorrhizal propagules are in an aqueous medium, Glomaline secretion is stimulated, which is present at high concentrations in the composition of this inoculant, conferring it special characteristics, speeding up this way, with its direct application, the processes of forming stable micro-aggregates in the soil, improving its physical structure immediately, and increasing oxygen levels, which enables a better colonization of this microorganism, guaranteeing an efficient mycorrhizal symbiosis development.

This inoculant promotes the direct application of the product in the soil, decreasing its volume when its concentration is increased, enabling this way its handling and transportation, and eliminating work.

It is mounted in a concentrated liquid support, with 8-months viability, which can be completely applied by means of fertigation systems, achieving a homogeneous distribution, checked by experiments at different scales. This fact has guaranteed a successful inoculation of all plants under the irrigation system, which is observed by significant yield increments (see technical report).

### References

- Dell Amico et al., Journal of Agricultural Science.. 2002
- Feldmann, F and ldczak, E. Inoculum production of Vesicular - arbuscular Mycorrhizal Fungi for use in tropical nurseries. In: Methods in Microbiology. Volume 24 ISBN 0-12-521524-x. Eds. Academic Press Limited. 1992
- Fernández, F. Chapter 3. Bases Cientifico Técnicas para el manejo de los sistemas micorrizados eficientemente. In: Rivera, Fernández, eds. INCA, La Habana, 2003.
- Fernández, F. et al., Patente 22641. OCPI. 1999
- Sieverding, E. Vesicular Arbuscular Mycorrhiza in Tropical Agrosystem. Deutsche Gesellsschaft fur techniische Zusammenarbeit (GTZ) GMBH, Federal Republic of Germany, 1991, p. 371.

## Claims

1. A liquid mycorrhizal inoculant **characterized in** the following composition:
a. Mycorrhizal propagules made up by resistance spores, arbuscular external mycelium, Glomaline and smaller than 40 um roots, colonized with the fungus in question.
b. Sorbitol, at a range between 5 and 10%.
c. Mannitol, at a range between 2 and 5%.
d. Tween 40, at a range between 5 and 7%.
e. Tween 80, at a range between 2 and 7%.
f. Polyethylene glycol applied at a range between 100 and 500 mg.l⁻¹.
g. Agar, at 0.1 %

2. A liquid mycorrhizal inoculant according to claim 1, **characterized in that** can be applied in water by means of irrigation and fertigation systems.

3. A liquid inoculant mycorrhizal according to claim 1, **characterized in that** it allows a concentration level of the order of 1 or 2 million spores.l⁻¹ and of 1 and 2 grams of mycelium.l⁻¹.

4. Polyethylene glycol according to claim 1, wherein the polyethylene glycol can be used with several molecular weights, at range of 4000 to 10000 with a solution concentration according to the molecular weight in a range of 100 to 500 mg.l-1.
